# EUROPEAN PATENT SPECIFICATION

(11) **EP 1 549 117 B1**
(45) Date of publication and mention of the grant of the patent: **17.02.2010**
(21) Application number: 04030475.0
(22) Date of filing: 22.12.2004
(51) Int. Cl.: H05K 1/00, H01R 9/03, A61B 8/12, A61B 1/00

(54) **Ultrasonic endoscope with ultrasonic signal cable connector device**
Ultraschallendoskop mit einem Ultraschallsignalkabelverbinder
Endoscope ultrasonique avec connecteur de câble pour les signaux ultrasonore

(30) Priority: 26.12.2003 JP 2003435620
(43) Date of publication of application: 29.06.2005
(73) Proprietor: Olympus Corporation, Tokyo 151-0072 (JP)
(72) Inventor: Saiga, Kazuya, Shibuya-ku, Tokyo (JP)
(74) Representative: von Hellfeld, Axel

(56) References cited:
- EP-A- 1 104 049
- US-A1- 2002 050 399
- PATENT ABSTRACTS OF JAPAN vol. 2000, no. 08, 6 October 2000 (2000-10-06) & JP 2000 139927 A (OLYMPUS OPTICAL CO LTD), 23 May 2000 (2000-05-23)

## Description

### BACKGROUND OF THE INVENTION

### 1. Field of the Invention

The present invention relates to an ultrasonic endoscope having an ultrasonic connector that connects to ultrasonic measuring equipment and observes the body cavity using ultrasonic wave, and to an ultrasonic signal cable connector device.

### 2. Description of the Related Art

Various types of endoscopes are widely used in the medical field, which are inserted into the body cavity to observe the living-body tissue or perform biopsy or treatment.

One type of such endoscopes is the ultrasonic endoscope. This ultrasonic endoscope has an ultrasonic transducer on the distal end of the insertion portion that is to be inserted in the body cavity. The ultrasonic transducer of this ultrasonic endoscope transmits ultrasonic wave to the living-body tissue, and receives the ultrasonic wave reflected from the living-body tissue. Thus, an ultrasonic tomographic image is generated by the various observation equipment connected to the ultrasonic endoscope, and the living-body tissue can be observed.

This ultrasonic endoscope is provided on the proximal end portion of the ultrasonic signal cable, as described in Japanese Patent Application Publication No. 2000-139927 for example, and has an ultrasonic connector that connects to an ultrasonic measuring equipment. This ultrasonic connector is provided with, for example, four Flexible Printed Circuits (hereafter "FPC"). The four FPCs each are connected to one end of each of multiple groups of signal lines. Further, the other end of each group of the multiple signal lines is connected to piezoelectric elements making up an electronic scanning ultrasonic transducer.

The FPCs and the multiple signal lines divided into groups are connected by the signal lines being soldered to contact pads provided on the FPCs.

Further, an FPC extension connecting pad is made for connecting an extension FPC. One end of this extension FPC can be inserted into the FPC connector of the ultrasonic connector and fixed. This FPC and the extension FPC are connected by the extension FPC connecting pad provided on the FPC being soldered to the pad provided on the extension FPC.

Further, the multiple signal lines extending from the ultrasonic transducer of the ultrasonic endoscope are divided into multiple groups, with an FPC disposed for each group.

A relatively large number, more than several dozen, of the above-described signals lines are built in, although this depends upon the number of piezoelectric elements. Therefore, the ultrasonic signal cable can have a narrower external diameter, the width of the multiple piezoelectric elements can be set narrow, and the electric connecting unit of the signal lines can also be set narrow, resulting in the much narrower diameter of the signal lines, as illustrated in the drawings of Japanese Patent Application Publication No. 2000-139927.

Further, the dimensions of the FPCs connected with the ultrasonic signal cable need to be smaller in diameter than that of the ultrasonic signal cable channel, in order to pass through the ultrasonic signal cable channel which has a small diameter, that is positioned in the insertion unit and so forth.

Therefore, the land width and the land spacing of the contact pad provided on the FPC is made to be very small. Accordingly, the assembly worker needs to have a high degree of skill for soldering the extremely fine signal lines to the contact pad for electric connection.

On the other hand, the FPC connector width and the FPC connector spacing of the multiple FPC connectors provided on the connecting connector of the ultrasonic connector are set relatively wide. Thus, the multiple FPC connectors are arranged so as to avoid contact failure. This ultrasonic connector has an extension FPC for ease of attaching to the connecting connector, and the extension FPC has a contact pad wherein the pad width and pad spacing is set so as to correspond to the FPC connector.

However, in the assembly process, the ultrasonic signal cables with FPCs connected beforehand are inserted through the ultrasonic signal cable channel, and also inserted through a cable insertion hole for the ultrasonic connector, following which extension FPCs are connected by soldering. Accordingly, the assembly worker needs to have a high degree of skill for the complicated soldering of FPCs and extension FPCs within the narrow ultrasonic connector, as well. Also, the soldered portions of the FPC and extension FPC have the problem of readily cracking under external force.

The present invention has been made in light of the above-described situation, and accordingly it is an object thereof to provide an ultrasonic signal cable connector to which are connected flexible wiring boards connected to ultrasonic signal cables which can be easily passed through the narrow ultrasonic signal cable channel, whereby electrical connection work within the narrow ultrasonic connector with little work space is not necessary, and also to provide an ultrasonic endoscope having this ultrasonic signal cable connector.

### SUMMARY OF THE INVENTION

According to the present invention, an ultrasonic endoscope according to claim 1 is provided.

### BRIEF DESCRIPTION OF THE DRAWINGS

Fig. 1 is a description diagram illustrating the configuration of an ultrasonic endoscope according to a first embodiment of the present invention;
Fig. 2 is a cross-sectional diagram illustrating the configuration of an ultrasonic connector according to the first embodiment of the present invention;
Fig. 3 is a plan view illustrating the configuration of an ultrasonic signal cable according to the first embodiment of the present invention;
Fig. 4 is a plan view illustrating the configuration of an ultrasonic signal cable connector device according to the first embodiment of the present invention;
Fig. 5 is a side view illustrating the configuration of an ultrasonic signal cable connector device according to the first embodiment of the present invention;
Fig. 6 is a side view illustrating the insertion method of the ultrasonic signal cable connector device into the ultrasonic signal cable channel according to the first embodiment relating to the present invention;
Fig. 7 is a plan view illustrating the configuration of an ultrasonic signal cable connector device according to a second embodiment of the present invention;
Fig. 8 is a side view illustrating the configuration of an ultrasonic signal cable connector device according to the second embodiment of the present invention;
Fig. 9 is a side view illustrating the insertion method of the ultrasonic signal cable connector device into the ultrasonic signal cable channel according to the second embodiment relating to the present invention.

### DETAILED DESCRIPTION OF THE PREFERRED EMBODIMENTS

### First Embodiment

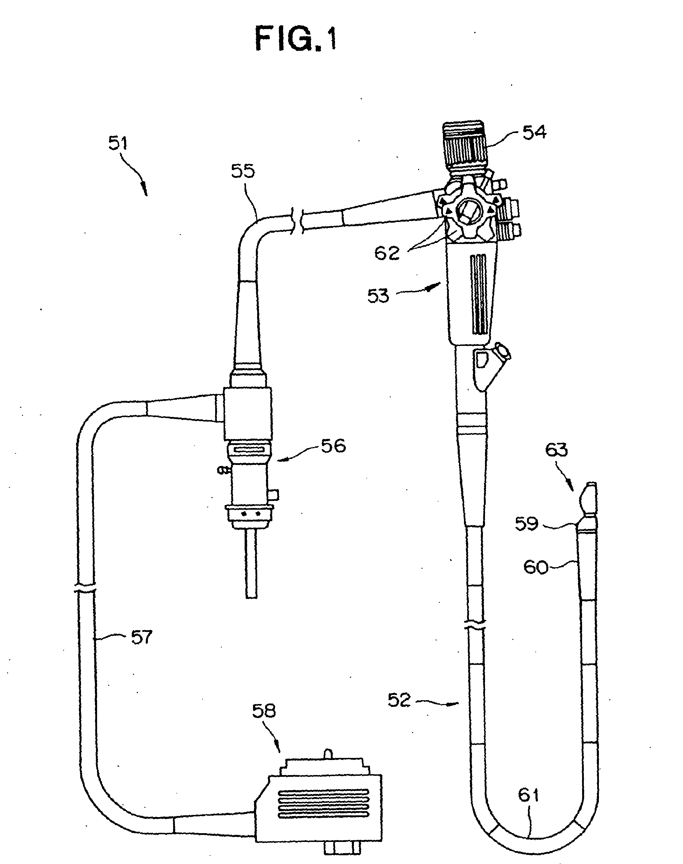

The configuration of the ultrasonic endoscope of the present invention will be described below based on the diagram, with reference to Fig. 1. An ultrasonic endoscope 51 primarily comprises an insertion portion 52 that is inserted into the body cavity, an operating unit 53 provided on the rear end of the insertion portion 52, an eyepiece 54 provided on the proximal end of the operating unit 53, a universal cord 55 extending from the operating unit 53, an endoscope connector 56 provided on the proximal end of the universal cord 55 that is connected to a light source device (not shown), and an ultrasonic signal cable 57 that extends from the endoscope connector 56 and has an ultrasonic connector 58 on the distal end thereof. Now, the ultrasonic connector 58 which is this connector unit is connected to an ultrasonic measuring equipment (not shown).

The insertion portion 52 is formed with a small diameter. The insertion portion 52 comprises, in order from the distal end, a hard distal-end portion 59, a bending portion 60, and a long flexible portion 61 that is capable of bending. An ultrasonic transducer unit 63 is located on the distal end side of the hard distal-end portion 59. An electronic scanning ultrasonic transducer, wherein multiple piezoelectric elements that send and receive ultrasonic wave are arrayed, is located on the ultrasonic transducer unit 63.

Disposed on the operating unit 53 are a bending knob 62, an air and water feed button, a suction button, an insertion opening for forceps treatment instruments, and so forth.

The bending knob 62 is an operating knob for bending the bending portion 60 of the insertion portion 52 in the desired direction. The air and water feed button is operated in the moment wherein air or water is fed to the air and water feeding channel provided within the insertion portion 52. The suction button is operated in the moment wherein air or water is suctioned into the suction channel. The insertion opening for forceps treatment instruments is an opening for the forceps channel through which forceps treatment instruments are inserted.

The optical image of the internal body cavity from the object lens provided on the hard distal-end portion 59 of the insertion portion 52 is guided by the image guide, and visually confirmed at the eyepiece 54.

The illumination light from the light source device wherein the endoscope connector 56 is connected is guided through the universal cord 55, and a light guide that is passed through the inside of the operating unit 53 and the insertion portion 52. Then the illumination light is emitted from the hard distal-end portion 59 onto the observation portions of the body cavity. The ultrasonic signal cable 57 that extends from the endoscope connector 56 is passed through the universal cord 55, and the ultrasonic signal cable channel (not shown) provided on the insertion portion 52 and the operating unit 53. One end of the ultrasonic signal cable 57 is connected to the electronic scanning ultrasonic transducer of the ultrasonic transducer unit 63, and the other end thereof is connected to the ultrasonic connector 58. The ultrasonic connector 58 is connected to the ultrasonic measuring equipment (not shown) that performs sending/receiving drive control of the ultrasonic wave from the electronic scanning ultrasonic transducer of ultrasonic transducer unit 63.

Further, the ultrasonic signal cable 57 is connected to the multiple piezoelectric elements of the electronic scanning ultrasonic transducer provided in the ultrasonic transducer unit 63. Multiple signal lines which are signal core lines, to be described below, that send/receive the ultrasonic signal from the piezoelectric elements, pass through the inside of the ultrasonic signal cable 57. These multiple signal lines are grouped according to a unit with a predetermined number of lines.

The one end sides of the multiple signal lines in the ultrasonic signal cable 57 are connected electrically to the multiple piezoelectric elements of the electronic scanning ultrasonic transducer provided on the ultrasonic transducer unit 63. The multiple signal lines are separated into multiple bundles as will be described below, and are inserted in the ultrasonic signal cable channel opening provided on the hard distal-end portion 59 of the insertion portion 52 from the other end side that is not connected with the piezoelectric elements. The ultrasonic signal cable 57 passes through the ultrasonic signal cable channel and is connected to the ultrasonic connector 58. Now, the ultrasonic signal cable channel is an insertion hole that is located within the bending portion 60 of the insertion portion 52, the flexible portion 61, the operating unit 53, the universal cord 55, and the endoscope connector 56.

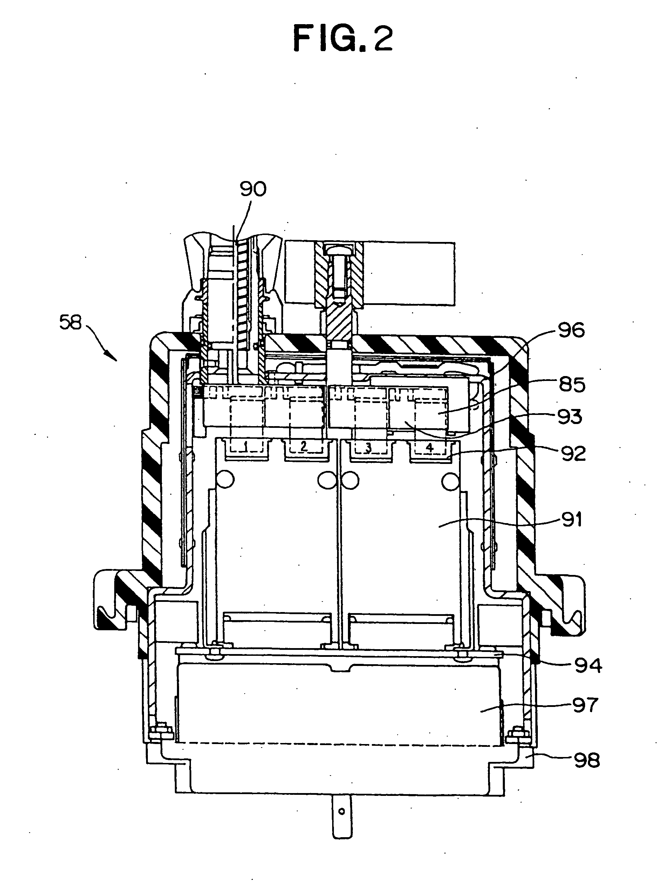

An ultrasonic signal cable connector device to be described below is provided on the distal end of the signal lines of each ultrasonic signal cable 57 for each group wherein the signal lines were grouped together. This ultrasonic signal cable connector device is connected to the connecting connector 92 (see Fig. 2) of the ultrasonic connector 58.

Next, the configuration of the ultrasonic connector 58 will be described, with reference to Fig. 2. Fig. 2 is a cross-sectional diagram of an ultrasonic connector.

The ultrasonic connector 58 mainly comprises a metal frame 96 in an approximately rectangular box shape, a cable insertion hole 90, multiple connecting connectors 92, four to be specific, an insulating sheet 93, multiple matching boards 91, two to be specific, a base board 94, a metal board 97, and a connector unit 98. The cable insertion hole 90 is an insertion hole for inserting the ultrasonic signal cable 57 into the metal frame 96. Multiple ultrasonic signal cable connector devices to be described below, which are provided on the distal end of the ultrasonic signal cable 57 that is inserted from the cable insertion hole 90, are attached to the connecting connectors 92. The ultrasonic signal cable connector devices are each attached to the connecting connectors 92, and in the moment wherein they are connected, the insulating sheet 93 is provided between the metal frame 96 and the ultrasonic signal cable connector device. In other words, the insulating sheet 93 insulates the connecting connectors 92 and the ultrasonic signal cable connector device in the moment that the ultrasonic signal cable connector device is attached and connected to the connecting connector 92. The matching boards 91 are electrically connected to the multiple connecting connectors 92. The matching boards 91 are boards on which matching circuits are formed. The matching circuits perform the matching between the ultrasonic measuring equipment and the electronic scanning ultrasonic transducer that is connected to the ultrasonic signal cable 57. The base board 94 is connected to the multiple matching boards 91. Further, the base board 94 has multiple connector pins for the purpose of connecting to the ultrasonic measuring equipment. The metal board 97 covers the area of the connector pin of the base board 94, and is fixed to the metal frame 96. In the moment that the ultrasonic connector 58 is connected to the ultrasonic measuring equipment, the connector unit 98 sets the metal frame 96 and the metal board 97 at the same electric potential as the reference potential of the ultrasonic measuring equipment.

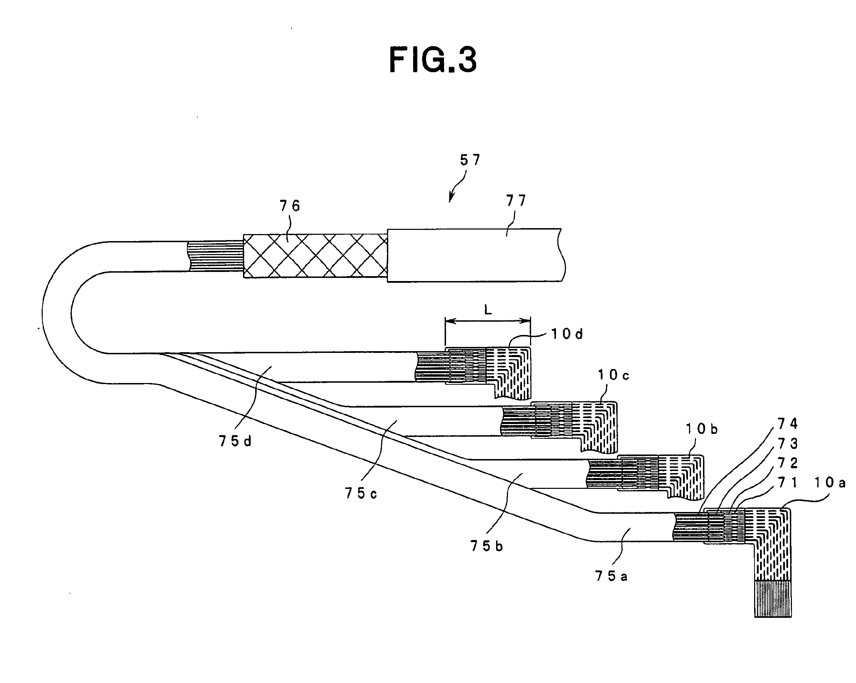

The configuration of the ultrasonic signal cable 57 that is connected to the ultrasonic connector 58 will be described with reference to Fig. 3. The ultrasonic signal cable 57 comprises an overall insulator covering 77, multiple coaxial lines 75a through 75d, and an overall shield 76 that bundles the coaxial lines 75a through 75d. Multiple signal core lines 71 are located within the coaxial lines 75a through 75d.

The multiple signal core lines 71 are each covered with an insulator layer 72. A shield 73 surrounds each of the insulator layers 72. An insulator covering layer 74 surrounds and covers the shields 73. In other words, inside of the coaxial lines 75a through 75d, the signal core lines 71 that are covered by the insulator layer 72, the shield 73 and the insulator covering layer 74 are grouped together with several lines in a unit.

On the distal ends of the multiple coaxial lines 75a through 75d, ultrasonic signal cable connector devices 10a through 10d that are connecting boards are each connected (hereafter, may on occasion be individually or collectively referred to as "ultrasonic signal cable connector device 10"). The ultrasonic signal cable connector devices 10a through 10d are connected, in order, along the length of the ultrasonic signal cable 57 on the distal ends of the coaxial lines 75a through 75d, each set apart only the length L of the ultrasonic signal cable connector device 10. In other words, the ultrasonic signal cable connector devices 10a through 10d are located in a perpendicular direction to the length of the ultrasonic signal cable 57, so as not to overlap the others.

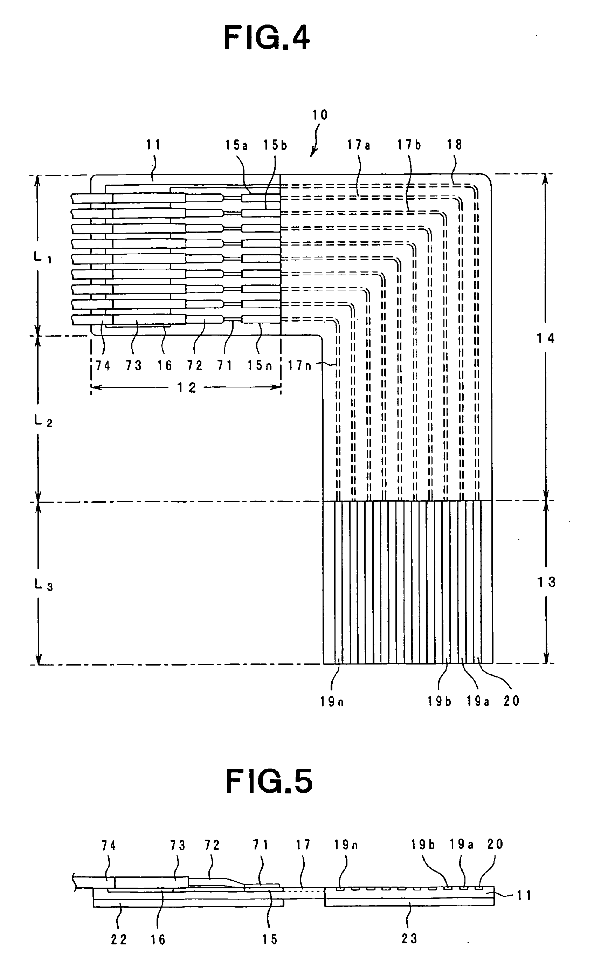

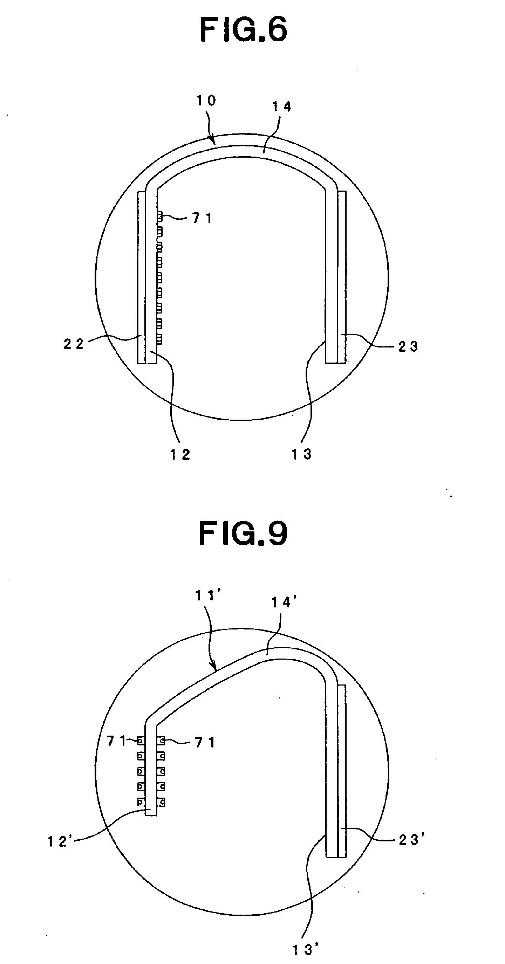

The ultrasonic signal cable connector device will be described below with reference to Figs. 4 through 6. Fig. 4 is a plan view illustrating the configuration of an ultrasonic signal cable connector device, Fig. 5 is a side view illustrating the configuration of an ultrasonic signal cable connector device, and Fig. 6 is a diagram illustrating an insertion method of inserting the ultrasonic signal cable connector device that is connected to the ultrasonic signal cable to the ultrasonic signal cable channel of the cable connector.

As illustrated in Fig. 4, the ultrasonic signal cable connector device (hereafter referred to as "cable connector") 10 has a flexible board 11 formed as one unit in an approximate L-shape. A connecting land and a circuit pattern are formed on the surface of the insulating base board of the flexible board 11.

On the surface of one edge of the L-shaped flexible board 11 is provided with multiple signal core line wiring lands 15a through 15n and grounding land 16, with equal spacing between. The wiring patterns 17a through 17n provided with equal spacing along the surface shape of the flexible board 11, and the grounding pattern 18, extend out from the signal core line wiring lands 15a through 15n and the grounding land 16.

Further, the signal core lines 71 of the coaxial lines 75a through 75d of the ultrasonic signal cable 57 are electrically connected by soldering to the multiple signal core line wiring lands 15a through 15n. The grounding land 16 is electrically connected by soldering to the shields 73 provided on the signal core lines 71.

On the surface of the other edge of the L-shaped flexible board 11 are provided with multiple connector connecting lands 19a through 19n and one connector connecting land 20, with equal spacing between. The other edge portion of the flexible board 11 wherein the connector connecting lands 19a through 19n and the connector connecting land 20 are provided is attached to the connecting connector 92 (see Fig. 2) of the ultrasonic connector 58. Thus, the flexible board 11 is electrically connected with the matching board 91. In this state, the connector connecting lands 19a through 19n are electrically connected to the wiring patterns 17a through 18n. Further, the connector connecting land 20 becomes connected with the grounding pattern 18.

The portion of the flexible board 11 wherein the signal core line lands 15a through 15n and the grounding land 16 are provided has a smaller dimension in the width direction (hereafter may be referred to as "length") L1 that is smaller than the internal diameter of the ultrasonic signal cable channel of the ultrasonic endoscope 51. Therefore, the land width of the signal core line lands 15a through 15n are approximately the same width as the diameter of the signal core line 71 of the ultrasonic signal cable 57. Now, the spacing of the signal core line lands 15a through 15n is set to be the dimensions of the spacing wherein the predetermined number of signal core lines 71 can be connected in the width direction L1.

Further, regarding the length of the flexible board 11 provided with the connector wiring lands 19a through 19n and the connector grounding land 20, the length L2 in the inserting direction to the connecting connector 92 of the ultrasonic connector 58 is approximately the same length as the length L1 of the flexible board 11 (L1 = L2), and is set to be less than the internal diameter of the ultrasonic signal cable channel.

The back face side of the flexible board 11 provided with the signal core line lands 15a through 15n and the ground land 16 has a first hard board portion 12 formed by adhering a hard member 22, as illustrated in Fig. 5. Further, on the back face of the flexible substrate 11 where the connector wiring lands 19a through 19n and the connector grounding land 20 are provided, a second hard board portion 13 is formed by adhering a hard member 23. The length L1 of the width dimension of the first hard board portion 12 and the length L2 of the width dimension of the second hard board portion 13 are formed to be approximately the equal length (L1 = L2) that is less than the internal diameter of the ultrasonic signal cable channel. Further, the length L3 of the length dimension of the flexible board 11 from the first hard board portion 12 to the second hard board portion 13 is less than the internal diameter of the ultrasonic signal cable channel, and is equal to the length L1 of the width dimension of the first hard board portion 12 and the length dimension L2 of the second hard board portion 13, or is less than that (L1 = L2 ≥ L3).

In other words, the L-shaped flexible board 11 has a first hard board portion 12 and a second hard board portion 13, wherein the first hard board portion 12 and the second hard board portion 13 are difficult to bend and deform. Therefore, the flexible board 11 has a soft board portion 14 that retains flexibility, in addition to having the first hard board portion 12 and the second hard board portion 13.

The signal core line lands 15a through 15n provided on the first hard board portion 12 are electrically connected to the signal core lines 71 of the ultrasonic signal cable 58 by using soldering devices for fine signal lines. Further, the grounding land 16 provided on the first hard board portion 12 is electrically connected to the shield 73 of the signal core line 71 by using soldering devices for fine signal lines.

Next, a method for inserting the ultrasonic signal cable 57 that is connected to the cable connector 10 from the hard distal-end portion 59 of the ultrasonic endoscope 51 through to the ultrasonic signal cable channel provided on the insertion portion 52, the operating unit 53, and universal cord 55, and the endoscope connector 56 will be described with reference to Fig. 6.

When viewed from the insertion direction, the cable connector 10 is bent in an approximately U-shape from the soft board portion 14 so that the face provided with the signal core line lands 15a through 15n and the grounding land 16 of the first hard board portion 12 and the face provided with the connector connecting lands 19a through 19n and the connector grounding land 20 of the second hard board portion 13 are facing each other.

As described above, the cable connector 10 is bent into a U-shape, and the assembly worker inserts this into the ultrasonic signal cable channel. At this time, the assembly worker can easily pass the article through the ultrasonic signal cable channel because the first and second hard board portions 12 and 13 are each formed less than the internal diameter of the ultrasonic signal cable channel. Now, in order to maintain the U-shape of the cable connector 10, the assembly worker may, for example, place a cap of a cylindrical tube or wrap cellophane tape around and pass it through into the ultrasonic signal cable channel.

Thus, the cable connector 10 at the distal end of the ultrasonic signal cable 57 that is passed through to the ultrasonic signal cable channel is inserted from the cable insertion hole 90 on the ultrasonic connector 58. Then, after the assembly worker has removed the cylindrical tube or cellophane tape that is maintaining the U-shaped bend of the cable connector 10, the second hard board portion 23 is attached to the connecting connector 92.

As described above, before the assembly worker inserts the ultrasonic signal cable 57 into the ultrasonic signal cable channel, the signal core line lands 15a through 15n and the grounding land 16 provided on the first hard board 12 of the cable connector 10 can be electrically connected to the signal core line 71 and the shield 73 of the ultrasonic signal cable 57. Accordingly, the assembly worker can perform the soldering operation for electrical connection with no obstructing structures. Therefore, since the soldering connecting work of the ultrasonic signal cable 57 and the cable connector 10 has no workspace limitation, the process becomes relatively simple. Further, the cable connector 10 is formed in an L-shape, and is formed as one unit with the second hard board portion 13 that is attached to the connecting connector 92 of the ultrasonic connector 58. Therefore, the soldering work within the ultrasonic connector 58 becomes unnecessary, and the L-shaped portion is less likely to tear even if external pressure is placed thereon.

### Second Embodiment

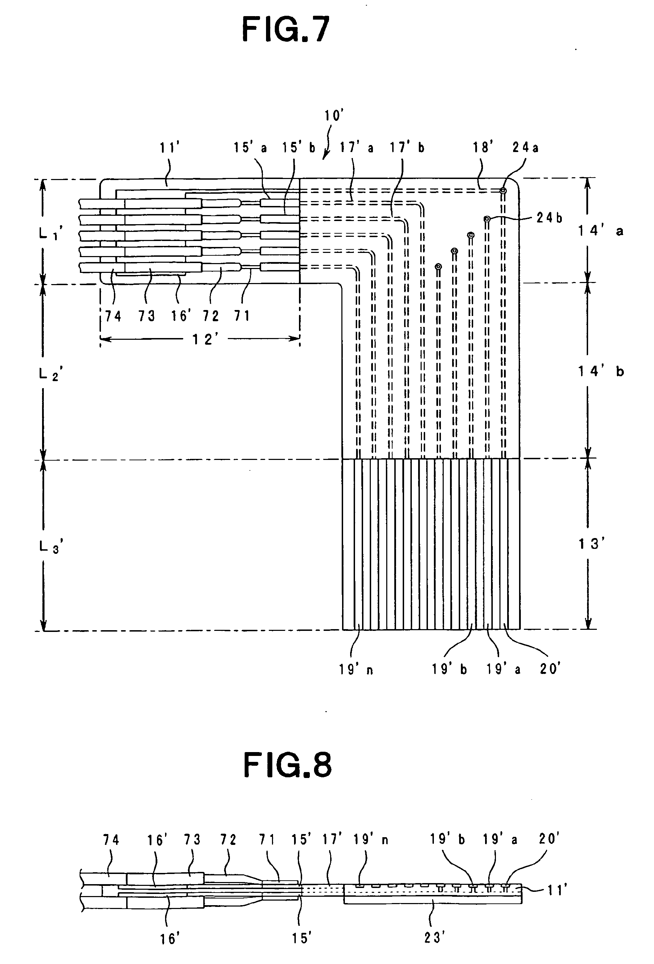

Next, the second embodiment of the ultrasonic signal cable connector device for an ultrasonic endoscope of the present invention will be described with reference to Figs. 7 through 9. Fig. 7 is a plan view illustrating the configuration of cable connector used for an ultrasonic signal cable that is the second embodiment of the ultrasonic signal cable connector device relating to the present invention, Fig. 8 is a side view thereof, and Fig. 9 is a side view illustrating the insertion method of the cable connector used as an ultrasonic signal cable into the ultrasonic signal cable channel that is the second embodiment of the ultrasonic signal cable connector device relating to the present invention. Now, components which are the same as those of the ultrasonic signal cable connector device illustrated in Figs. 4 and 5 will be denoted with the same reference numerals, and description thereof will be omitted.

The cable connector 10' of the second embodiment has the signal core line lands 15'a, 15'b, and so on, and the grounding land 16' on one edge of both the front face and the back face of the L-shaped flexible board 11'. From the signal core line lands 15'a, 15'b, and so on, and the grounding land 16' of the two faces front and back, the wiring pattern 17'a, 17'b and so on and grounding pattern 18' is extended in the lengthwise direction. Further, the through holes 24a, 24b, and so on, are provided on the L-shape form of the flexible board 11 where the shape changes. The wiring pattern 17'a, 17'b, and so on, and the grounding pattern 18' provided on the back face of the flexible board 11 are drawn through to the front face side via the through holes 24a, 24b, and so on.

On the front face of the other edge of the flexible board 11, the connector connecting lands 19'a through 19'n for attaching to the connecting connector 92 of the ultrasonic connector 58, and the connector grounding land 20' are provided. The connector connecting lands 19'a through 19'n and the aforementioned connector grounding lands 20' are connected to the wiring patterns 17'a, 17'b, and so on, and the through holes 24a, 24b, and so on.

In other words, the one edge of the flexible board 11 has a certain amount of strength because the signal core line lands 15'a, 15'b, and so on, and the grounding land 16', are each provided on the front and back faces thereof. Further, the strength of the one edge of the flexible board 11 is increased because the signal core lines 71 and the shields 73 of the ultrasonic signal cable 57 are soldered and connected with the signal core line lands 15'a, 15'b, and so on, and the grounding land 16'. Therefore, the first hard board portion 12' can be formed on the flexible board 11 without adhering the hard member 22 (see Fig. 5) as described in the first embodiment.

Further, in the case that the number of the signal core lines 71 of the ultrasonic signal cable 57 that connects to the cable connector 10' is the same as the above-described first embodiment, by providing the signal core line lands 15'a, 15'b, and so on, and the grounding land 16', on both the front and back faces of the first hard board portion 12', the length L1' of the width direction measurement of the first hard board portion 12' becomes about half of that of the length L1 of the first hard board 12 of the first embodiment (L1' = L1/2).

Further, the wiring patterns 17'a, 17'b, and so on, and the grounding pattern 18' from the signal core line lands 15'a, 15'b, and so on, and the grounding land 16 on both the front and back of the first hard board portion 12' are extended straight out in the lengthwise direction of the first hard board portion 12'. Further, within the portion of the L-shape that change shape on the flexible board 11', the wiring patterns 17'a, 17'b, and so on, and the grounding pattern 18' on the back face side are drawn out to the front face side by the through holes 24a, 24b, and so on. Also, all of the wiring patterns 17'a, 17'b, and so on, and the grounding pattern 18' extend straight out to the front face of the flexible board 11' towards the second hard board portion 13' wherein the hard member 23' is adhered similar to the above-described first embodiment. The extended edges of all of the wiring patterns 17'a, 17'b, and so on and the grounding pattern 18' are connected to the connector connecting lands 19'a through 19'n and the connector grounding land 20. As a result, the lengthwise measurement and the width dimension of the second hard unit 13' can be made to match the measurements of the connecting connector 92 of the aforementioned ultrasonic connector 58 similar to the above-described first embodiment.

Thus, the first hard board portion 12 is formed on the cable connector 10' by soldering and connecting the signal core line lands 15'a through 15'n and the grounding land 16' provided on the front and back faces of the L-shaped flexible board 11' with the signal core lines 71 and the shields 73 of the ultrasonic signal cable 57. Further, the second hard board portion 13' wherein the hard member 23 is adhered to the back face formed with the connector connecting lands 19'a through 19'n and the connector grounding land 20' is formed on the cable connector 10'. Therefore, the first hard board portion 12' and the second hard board portion 13' of the cable connector 10' do not readily bend. In other words, the flexible board 11 has a flexible board 14 that retains the flexibility other than the first hard board 12 and the second hard board 13.

The signal core line lands 15'a, 15'b, and so on, provided on both the front and back faces of the first hard board portion 12' on the cable connector 10' are electrically connected with the signal core lines 71 of the ultrasonic signal cable 58, by using a soldering device for fine signal lines. Further, the grounding land 16' is electrically connected with shields 73 of the signal core lines 71 by using a soldering device for fine signal lines.

Next, a method for inserting the ultrasonic signal cable 57 that is connected to the cable connector 10' from the hard distal-end portion 59 of the ultrasonic endoscope 51 through to the ultrasonic signal cable channel provided on the insertion portion 52, the operating unit 53, and universal cord 55, and the endoscope connector 56 will be described with reference to Fig. 9.

As illustrated in Fig. 9, when viewed from the insertion direction, the cable connector 10', which is electrically connected with the distal end of the ultrasonic signal cable 57, is bent in an approximate U-shape at the soft board portion 14' so that the face provided with the signal core line lands 15'a, 15'b, and so on, and the grounding land 16, of the first hard board portion 12' and the face provided with the connector connecting lands 19'a through 19'n and the connector grounding land 20' of the second hard board portion 13' are facing each other.

The cable connector 10' is bent into a U-shape, and the assembly worker inserts this into the ultrasonic signal cable channel. At this time, the assembly worker can easily pass it through the ultrasonic signal cable channel because the first and second hard board portions 12' and 13' are each set to be less than the internal diameter of the ultrasonic signal cable channel. Now, in order to maintain the U-shape of the cable connector 10', the assembly worker may, for example, place a cap of a cylindrical tube or wrap cellophane tape around and pass it through into the ultrasonic signal cable channel.

Thus, the cable connector 10' at the distal end of the ultrasonic signal cable 57 that is passed through to the ultrasonic signal cable channel is inserted from the cable insertion hole 90 on the ultrasonic connector 58. Then, after the assembly worker has removed the cylindrical tube or cellophane tape that is maintaining the U-shaped bend of the cable connector 10', the second hard board portion 23' is attached to the connecting connector 92.

According to the second embodiment, the width dimension L1' of the first hard board portion 12' can be set to approximately half (L1' = L1/2) of the width dimension L1 of the first hard board portion 12 according to the first embodiment, and therefore the length dimension L2' of the second hard board portion 13' can be a value relatively close to the diameter of the ultrasonic signal cable channel 25.

Further, the wiring pattern 17'a through 17'n and the grounding pattern 18' and the through holes 24a, 24b, and so on that extend directly from both the front and back face of the first hard board 12' can have the hard member adhered to and the third hard board portion 14'a formed on either the front or back face of a portion that forms the L-shaped portion. By forming the third hard board portion 14'a, the through holes 24a, 24b, and so on are protected from stress due to the bending and changing shape. Now, a flexible board portion 14'b is formed that maintains the flexibility of the flexible board 11', on the flexible board 11', other than where the first hard board portion 12', the second hard board portion 13', and the third hard board portion 14'a are formed.

As described above, before the assembly worker inserts the ultrasonic signal cable 57 into the ultrasonic signal cable channel, the signal core line lands 15'a through 15'n and the grounding land 16' provided on the first hard board 12 of the cable connector 10' can be electrically connected to the signal core line 71 and the shield 73 of the ultrasonic signal cable 57. Therefore, the assembly worker can perform the soldering operation for electrical connection with no obstructing structures. Therefore, since the soldering connecting work of the ultrasonic signal cable 57 and the cable connector 10' has no limitation in the connecting specialized equipment or work environment, the process becomes relatively simple. Further, the cable connector 10' is formed in an L-shape, and is formed as one unit with the second hard board portion 13 that is attached to the connecting connector 92 of the ultrasonic connector 58. Therefore, the soldering work within the ultrasonic connector 58 becomes unnecessary, and the L-shaped portion of the cable connector 10' becomes more resistant to breakage pressure.

Having described the preferred embodiments of the invention referring to the accompanying drawings, it should be understood that the present invention is not limited to those precise embodiments and various changes and modifications hereof could be made by one skilled in the art without departing from the scope of the invention as defined in the appended claims.

## Claims

1. An ultrasonic endoscope (51) comprising:
an insertion portion (52) to be inserted in a body cavity;
an ultrasonic transducer (63) provided on a distal end of the insertion portion (52) to be inserted in a body cavity, for generating ultrasonic wave to the tissue of the body cavity and receiving the ultrasonic wave reflected from the body cavity tissue so as to generate an ultrasonic signal;
an ultrasonic signal cable (57) having a plurality of signal lines (71) connected wherein one end is connected to a plurality of piezoelectric elements making up the ultrasonic transducer (63), and the other end is connected to an ultrasonic signal cable connector device (10);
an ultrasonic connector (58) connected to the ultrasonic signal cable (57) and to ultrasonic measuring equipment;
the ultrasonic signal cable connector device (10) comprising:
a flexible wiring board (11) formed in an approximate L-shape with a wiring pattern (17a ... 17n) for connecting the plurality of signal lines (71) of the ultrasonic signal cable (57) to the ultrasonic connector (58);
a first hard board portion (12) wherein a plurality of wiring lands (15a ... 15n) for connecting the plurality of signal lines (71) of the ultrasonic signal cable (57) to the wiring pattern (17a ... 17n) are formed on one face of the flexible wiring board (11), wherein the portion where the wiring lands (15a ... 15n) are formed is hardened; and
a second hard board portion (13) wherein a plurality of connector connecting lands (19a ... 19n) for connecting the ultrasonic connector (58) to the wiring pattern (17a ... 17n) are formed on the other face of the flexible wiring board (11), wherein the portion where the connector connecting lands (19a ... 19n) are formed is hardened;
wherein the L-shaped flexible wiring board (11) is capable of being bent in an approximate U-shape such that the first hard board portion (12) and the second hard board portion (13) face each other.

2. An ultrasonic endoscope according to Claim 1, wherein the plurality of wiring lands (15a ... 15n) for connecting the signal lines (71) of the ultrasonic signal cable (57) are formed on the front and back faces of the first hard board portion (12);
and wherein the plurality of wiring lands (15a ... 15n) formed on the back face of the first hard board portion (12) are connected to the plurality of connector connecting lands (19a ... 19n) of the second hard board portion (13), via a wiring pattern (17a', 17b') that has through holes (24a, 24b) provided on the flexible wiring board (11).

3. An ultrasonic endoscope according to either Claim 1 or Claim 2, wherein the dimensions in the width direction of the first hard board portion (12) and the length direction of the second hard board portion (13) are less than the inner diameter of the ultrasonic signal cable (57) channel provided within the insertion portion (52) of the ultrasonic endoscope (51).

## Patentansprüche

1. Ultraschall-Endoskop (51) umfassend:
einen Einführabschnitt (52), der in eine Körperöffnung einzuführen ist;
einen Ultraschallwandler (63), der an einem distalen Ende des in eine Körperöffnung einzuführenden Einführabschnitts (52) vorgesehen ist, zum Erzeugen einer Ultraschallwelle an dem Gewebe der Körperöffnung und zum Empfangen der von dem Körperöffnungs-Gewebe reflektierten Ultraschallwelle, um ein Ultraschallsignal zu erzeugen;
ein Ultraschall-Signalkabel (57), das eine Vielzahl von verbundenen Signalleitungen (71) aufweist, wobei ein Ende mit einer Vielzahl von piezoelektrischen Elementen verbunden ist, die den Ultraschallwandler (63) bilden, und das andere Ende mit einem Ultraschall-Signalkabel-Anschlussgerät (10) verbunden ist;
ein Ultraschall-Anschluss (58), der mit dem Ultraschall-Signalkabel (57) und mit Ultraschall-Messausrüstung verbunden ist;
wobei das Ultraschall-Signalkabel-Anschlussgerät (10) umfasst:
eine flexible Leiterplatte (11), die in einer annähernden L-Form ausgebildet ist, mit einem Leitermuster (17a ... 17n) zum Verbinden der Vielzahl von Signalleitungen (71) des Ultraschall-Signalkabels (57) mit dem Ultraschall-Anschluss (58);
einen ersten festen Platinenabschnitt (12), wobei eine Vielzahl von Anschlussflecken (15a ... 15n) zum Verbinden der Vielzahl von Signalleitungen (71) des Ultraschall-Signalkabels (57) mit dem Leitermuster (17a ... 17n) an einer Seite der flexiblen Leiterplatte (11) ausgebildet ist, wobei der Abschnitt, in dem die Anschlussflecken (15a ... 15n) ausgebildet sind, gehärtet ist; und
einen zweiten festen Platinenabschnitt (13), wobei eine Vielzahl von Anschluss-Verbindungskontaktflecken (19a ... 19n) zum Verbinden des Ultraschall-Anschlusses (58) mit dem Leitermuster (17a ... 17n) auf der anderen Seite der flexiblen Leiterplatte (11) ausgebildet ist, wobei der Abschnitt, in dem die Anschluss-Verbindungskontaktflecken (19a ... 19n) ausgebildet sind, gehärtet ist;
wobei die L-förmige flexible Leiterplatte (11) dazu geeignet ist, in eine annähernde U-Form derart gebogen zu werden, dass der erste feste Platinenabschnitt (12) und der zweite feste Platinenabschnitt (13) sich gegenüberliegen.

2. Ultraschall-Endoskop nach Anspruch 1, wobei die Vielzahl von Anschlussflecken (15a ... 15n) zum Verbinden der Signalleitungen (71) des Ultraschall-Signalkabels (57) auf der Vorder- und der Rückseite des ersten festen Platinenabschnitts (12) ausgebildet sind;
und wobei die Vielzahl von Anschlussflecken (15a ... 15n), die auf der Rückseite des ersten festen Platinenabschnitts (12) ausgebildet sind, mit der Vielzahl von Anschluss-Verbindungskontaktflecken (19a ... 19n) des zweiten festen Platinenabschnitts (13) über ein Leitermuster (17a', 17b') verbunden sind, das Durchgangslöcher (24a, 24b) aufweist, die auf der flexiblen Leiterplatte (11) vorgesehen sind.

3. Ultraschall-Endoskop nach entweder Anspruch 1 oder Anspruch 2, wobei die Dimensionen in die Breitenrichtung des ersten festen Platinenabschnitts (12) und in die Längsrichtung des zweiten festen Platinenabschnitts (13) geringer sind als der Innendurchmesser des Ultraschall-Signalkabel (57)-Kanals, der innerhalb des Einführabschnitts (52) des Ultraschall-Endoskops (51) vorgesehen ist.

## Revendications

1. Endoscope à ultrasons (51) comprenant :
une partie d'insertion (52) à insérer dans une cavité du corps ;
un transducteur à ultrasons (63) prévu sur une extrémité distale de la partie d'insertion (52) à insérer dans une cavité du corps, pour émettre une onde ultrasonore vers le tissu de la cavité du corps et recevoir l'onde ultrasonore réfléchie à partir du tissu de la cavité du corps, de manière à produire un signal ultrasonore ;
un câble pour signaux ultrasonores (57) ayant une pluralité de lignes de signaux (71) connectées, dans lequel une extrémité est connectée à une pluralité d'éléments piézoélectriques composant le transducteur à ultrasons (63) et l'autre extrémité est connectée à un dispositif formant connecteur de câble pour signaux ultrasonores (10) ;
un connecteur à ultrasons (58) connecté au câble pour signaux ultrasonores (57) et au matériel de mesure à ultrasons ;
le dispositif formant connecteur de câble pour signaux ultrasonores (10) comprenant :
un tableau de connexions souple (11) formé sensiblement en une forme de L avec un motif de câblage (17a...17n) destiné à connecter la pluralité de lignes de signaux (71) du câble pour signaux ultrasonores (57) au connecteur à ultrasons (58);
une première partie de carte dure (12) dans laquelle une pluralité de plages de raccordement (15a...15n) destinées à connecter la pluralité de lignes de signaux (71) du câble pour signaux ultrasonores (57) au motif de câblage (17a...17n) est formée sur une face du tableau de connexions souple (11), dans lequel la partie où les plages de raccordement (15a...15n) sont formées, est durcie ; et
une seconde partie de carte dure (13) dans laquelle une pluralité de plages de connexion du connecteur (19a...19n) destinées à connecter le connecteur à ultrasons (58) au motif de câblage (17a...17n) est formée sur l'autre face du tableau de connexions souple (11), dans lequel la partie où les plages de connexion du connecteur (19a...19n) sont formées, est durcie ;
dans lequel le tableau de connexions souple (11) en forme de L est apte à être plié sensiblement en forme de U de telle sorte que la première partie de carte dure (12) et la seconde partie de carte dure (13) se fassent face l'une à l'autre.

2. Endoscope à ultrasons selon la revendication 1,
dans lequel la pluralité de plages de raccordement (15a...15n) destinées à connecter les lignes de signaux (71) du câble pour signaux ultrasonores (57) est formée sur les faces avant et arrière de la première partie de carte dure (12) ;
et dans lequel la pluralité de plages de raccordement (15a...15n) formée sur la face arrière de la première partie de carte dure (12) est connectée à la pluralité de plages de connexion du connecteur (19a...19n) de la seconde partie de carte dure (13), via un motif de câblage (17a', 17b') qui comporte des trous traversants (24a, 24b) ménagés sur le tableau de connexions souple (11).

3. Endoscope à ultrasons selon l'une quelconque des revendications 1 ou 2, dans lequel les dimensions dans le sens de la largeur de la première partie de carte dure (12) et dans le sens de la longueur de la seconde partie de carte dure (13) sont inférieures au diamètre intérieur du canal du câble pour signaux ultrasonores (57) ménagé à l'intérieur de la partie d'insertion (52) de l'endoscope à ultrasons.
